# EUROPEAN PATENT APPLICATION

(11) **EP 4 248 892 A1**
(43) Date of publication of application: **27.09.2023**
(21) Application number: 23163337.1
(22) Date of filing: 22.03.2023
(51) Int. Cl.: A61B 18/08, A61B 18/14, A61B 18/00

(54) **MULTI-SENSOR ABLATION PROBE WITH TREATMENT ELECTRODE**

(30) Priority: 22.03.2022 US 202263269739 P
(71) Applicant: DIXI Neurolab, Inc., Oxford, MI 48371 (US)
(72) Inventor: GALE. John Thomas, Myrtle Beach, SC29577 (US)
(74) Representative: DTS Patent- und Rechtsanwälte Schnekenbühl und Partner mbB

(57) **Abstract**

An ablation probe includes an elongated body having a tapered distal end and a proximal end that are aligned along an axis; first and second electrical sensors disposed on the elongated body; and an electrode disposed on the elongated body between the first and second electrical sensors. A power source is electrically coupled to the electrical sensors and to the electrode. A computer has an input electrically coupled to the electrical sensors to receive first and second output signals, respectively, from the electrical sensors. A non-transitory computer-readable memory is operatively coupled to the computer and stores computer-readable instructions that, when executed by the computer, cause the computer to: analyze an electrical characteristic measured by the first and second electrical sensors, and produce an output control signal when the electrical characteristic measured by the first and second electrical sensors indicates that the electrode is aligned with a target anatomical feature.

## Description

### Cross-Reference to Related Applications

This application claims priority to U.S. Provisional Application No. 63/269,739, titled "Multi-Sensor Probe with Treatment Electrode," filed on March 22, 2022, which is hereby incorporated by reference.

### Technical Field

This application relates generally to electrodes and methods for the precise localization of tissue.

### Background

Chronic back pain affects millions of people. Treatment approaches include spinal decompression, medication, physical therapy, and often in combination. In severe and persistent cases, chronic back pain treatments can be expanded to neuromodulation and/or tissue destructive procedures aimed at the spinal nerves, dorsal root ganglia (DRG), that carry the pain impulses to the brain.

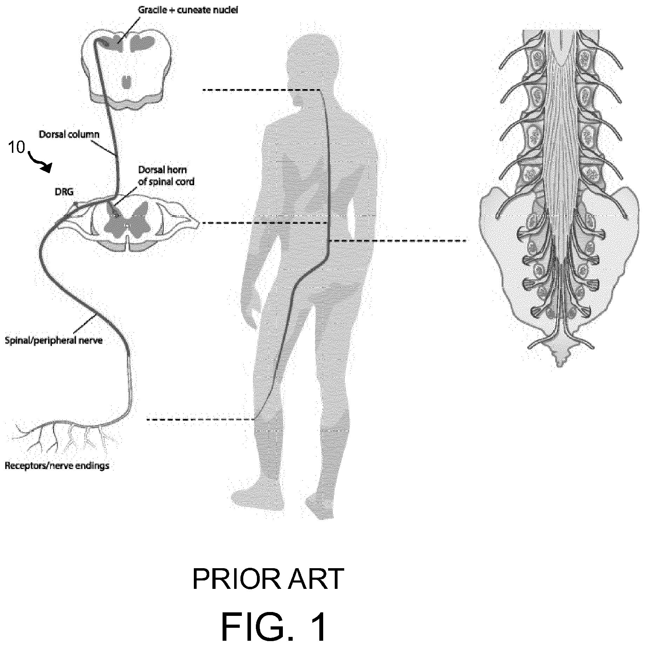

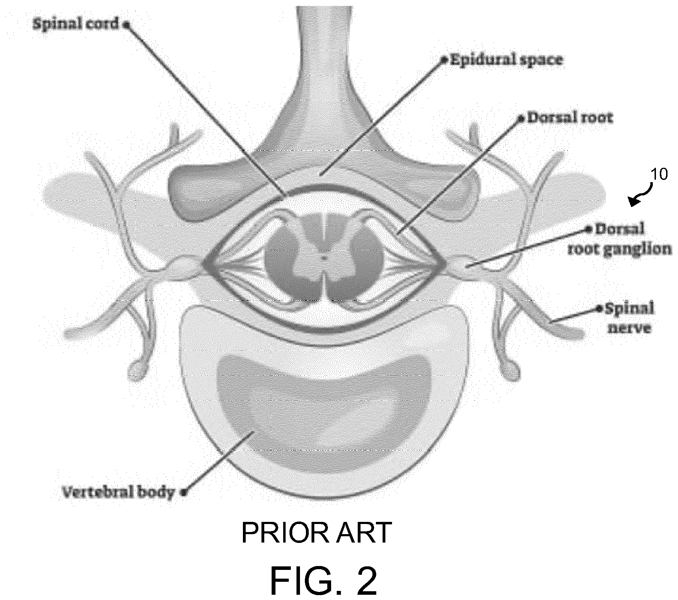

A recent focus of treating chronic back pain is the dorsal root ganglia (DRG) 10, which is illustrated schematically in **Fig. 1****.** A detailed view of the DRG 10 is illustrated in **Fig. 2****.**

### Summary

Example embodiments described herein have innovative features, no single one of which is indispensable or solely responsible for their desirable attributes. The following description and drawings set forth certain illustrative implementations of the disclosure in detail, which are indicative of several exemplary ways in which the various principles of the disclosure may be carried out. The illustrative examples, however, are not exhaustive of the many possible embodiments of the disclosure. Without limiting the scope of the claims, some of the advantageous features will now be summarized. Other objects, advantages and novel features of the disclosure will be set forth in the following detailed description of the disclosure when considered in conjunction with the drawings, which are intended to illustrate, not limit, the invention.

An aspect of the invention is directed to an ablation system. The ablation system includes an ablation probe that comprises an elongated body having a tapered distal end and a proximal end that are aligned along an axis; first and second electrical sensors disposed on the elongated body; and an electrode disposed on the elongated body between the first and second electrical sensors. The ablation system further includes a power source electrically coupled to the first and second electrical sensors and to the electrode; a computer having an input electrically coupled to the first and second electrical sensors to receive first and second output signals, respectively, from the first and second electrical sensors; and a non-transitory computer-readable memory operatively coupled to the computer. The non-transitory computer-readable memory stores computer-readable instructions that, when executed by the computer, causes the computer to: analyze an electrical characteristic measured by the first and second electrical sensors, and produce an output control signal when the electrical characteristic measured by the first and second electrical sensors indicates that the electrode is aligned with a target anatomical feature.

In one or more embodiments, the ablation system further comprises an interlock switch electrically connected to the electrode, the power source, and the computer, wherein the interlock switch has a default open state in which the electrode is electrically disconnected from the power source and a closed state in which the electrode is electrically connected to the power source, the interlock switch configured to transition from the default open state to the closed state in response to the output control signal.

In one or more embodiments, the electrode is aligned with the target anatomical feature when the electrical characteristic measured by the first and second electrical sensors has about the same magnitude. In one or more embodiments, the electrode is aligned with the target anatomical feature when the electrical characteristic measured by the first and second electrical sensors indicates that the first and second electrical sensors are in physical contact with the target anatomical feature. In one or more embodiments, the electrode is aligned with the target anatomical feature when the magnitude of the electrical characteristic measured by the first and second electrical sensors is higher than a respective baseline magnitude.

In one or more embodiments, the first electrical sensor is located closer to the tapered distal end than the second electrical sensor, and the electrode is aligned with the target anatomical feature when the electrical characteristic measured by the first electrical sensor indicates that the first electrical sensor has passed over the target anatomical feature and the electrical characteristic measured by the second electrical sensor indicates that the second electrical sensor is not in physical contact with the target anatomical feature. In one or more embodiments, the electrical characteristic comprises a resistance or an impedance.

In one or more embodiments, the ablation system further comprises a thermocouple disposed on the elongated body adjacent to the electrode. In one or more embodiments, the first and second electrical sensors comprise band contacts that extend along at least a portion of a perimeter of the elongated body. In one or more embodiments, the band contacts comprise ring contacts that form a respective loop along the perimeter of the elongated body. In one or more embodiments, the first and second electrical sensors and the electrode are aligned with respect to the axis.

Another aspect of the invention is directed to a multi-electrode ablation system. The multi-electrode ablation system comprises a multi-electrode ablation probe that includes an elongated body having a tapered distal end and a proximal end that are aligned along an axis; a plurality of electrical sensors disposed on the elongated body; and a plurality of electrodes disposed on the elongated body, the electrodes and electrical sensors having an alternating arrangement in which each electrode is located between a respective neighboring pair of electrical sensors. The multi-electrode ablation system further comprises one or more power sources electrically coupled to the electrical sensors and the electrodes; a computer having an input electrically coupled to the electrical sensors to receive respective output signals from the electrical sensors; and a non-transitory computer-readable memory operatively coupled to the computer. The non-transitory computer-readable memory stores computer-readable instructions that, when executed by the computer, causes the computer to: analyze an electrical characteristic measured by the electrical sensors, and produce an output control signal when the electrical characteristic measured by the electrical sensors indicates that the electrodes are aligned with a target anatomical feature.

Another aspect of the invention is directed to a method of ablating a mammalian subject, comprising inserting an ablation probe into a mammalian subject. The ablation probe comprises an elongated body having a tapered distal end and a proximal end that are aligned along an axis; first and second electrical sensors disposed on the elongated body; and an electrode disposed on the elongated body between the first and second electrical sensors, wherein: the first and second electrical sensors and the electrode are electrically coupled to a power source, and the first and second electrical sensors have outputs that are electrically coupled to an input of a computer. The method further comprises monitoring, with the computer, an electrical characteristic measured by the first and second electrical sensors; producing, with the computer, an output control signal when the electrical characteristic measured by the first and second electrical sensors indicates that the electrode is aligned with a target anatomical feature; and ablating the target anatomical feature, with the electrode, after the electrode is aligned with the target anatomical feature.

In one or more embodiments, the method further comprises automatically applying power to the electrode in response to the output control signal. In one or more embodiments, the method further comprises automatically closing an interlock switch in response to the output control signal, the interlock switch electrically connecting the electrode to a power source when the interlock switch is in a closed state.

In one or more embodiments, the method further comprises determining, with the computer, when a magnitude of the electrical characteristic measured by the first and second electrical sensors is higher than the magnitude of a respective baseline output signal of the first and second electrical sensors; and producing the output control signal when the magnitude of the electrical characteristic measured by the first and second electrical sensors is higher than the magnitude of the respective baseline output signal of the first and second electrical sensors.

In one or more embodiments, the method further comprises (a) determining, with the computer, when a magnitude of the electrical characteristic measured by the first electrical sensor is higher than the magnitude of a baseline output signal of the first electrical sensor and the magnitude of the electrical characteristic measured by the second electrical sensor is about equal to the magnitude of a baseline output signal of the second electrical sensor; (b) after step (a), determining, with the computer, when the magnitude of the electrical characteristic measured by the first and second electrical sensors is about equal to the magnitude of the baseline output signals of the first and second electrical sensors, respectively; and (c) producing the output control signal when the magnitude of the electrical characteristic measured by the first and second electrical sensors is about equal to the magnitude of the baseline output signals of the first and second electrical sensors, respectively, wherein the first electrical sensor is located closer to the tapered distal end than the second electrical sensor.

In one or more embodiments, the method further comprises (d) advancing the ablation probe distally in the mammalian subject between steps (a) and (b), wherein the ablation probe is in a first position in step (a) and in a second position in step (b). In one or more embodiments, the method further comprises (e) determining, with the computer, when the magnitude of the electrical characteristic measured by the second electrical sensor is higher than the magnitude of the baseline output signal of the second electrical sensor and the magnitude of the electrical characteristic measured by the first electrical sensor is about equal to the magnitude of the baseline output signal of the first electrical sensor; (f) advancing the ablation probe distally in the mammalian subject between steps (b) and (e), wherein the ablation probe is in a third position in step (e), the second position between the first position and the third position; and (g) retracting the ablation probe proximally in the mammalian subject after step (e) to a fourth position, the fourth position between the first position and the third position; (h) after step (g), repeating step (b), wherein step (c) occurs after step (h).

In one or more embodiments, the electrical characteristic comprises a resistance or an impedance. In one or more embodiments, the method further comprises producing a sensory output signal, with the computer, in response to the output control signal. In one or more embodiments, the method further comprises monitoring a measured temperature of the target anatomical feature with a thermocouple on the ablation probe, the thermocouple in electrical communication with the computer; and stopping an ablation of the target anatomical feature when the measured temperature is higher thana predetermined ablation threshold temperature for a predetermined time period.

### Brief Description of the Drawings

For a fuller understanding of the nature and advantages of the concepts disclosed herein, reference is made to the detailed description of preferred embodiments and the accompanying drawings.
Figs. 1 and 2 illustrate the dorsal root ganglia (DRG) according to the prior art.
Fig. 3 is a simplified view of an ablation system according to an embodiment with an interlock switch in an open state.
Fig. 4 is a simplified view of the ablation system illustrated Fig. 3 with the interlock switch in a closed state.
Fig. 5 is a simplified view of an ablation system according to another embodiment.
Fig. 6 is a simplified view of an ablation system according to another embodiment.
Fig. 7 is a simplified view of positioning an ablation probe with respect to the DRG according to an embodiment.
Fig. 8 is an example graph of a measured electrical characteristic versus time as the ablation probe is positioned with respect to the DRG, according to the embodiment illustrated in Fig. 7.
Fig. 9 is a simplified view of positioning an ablation probe with respect to the DRG according to another embodiment.
Fig. 10 is an example graph of a measured electrical characteristic versus time as the ablation probe is positioned with respect to the DRG, according to the embodiment illustrated in Fig. 9.
Fig. 11 is a simplified view a multi-electrode ablation probe according to an embodiment.
Fig. 12 is a simplified view of positioning the multi-electrode ablation probe illustrated in Fig. 11 with respect to the DRG according to an embodiment.
Fig. 13 is a simplified view a multi-electrode ablation probe according to another embodiment.
Fig. 14 is a flow chart of a method for locating and damaging a target anatomical feature according to an embodiment.
Figs. 15A and 15B illustrates an ablation probe in first and second positions, respectively, according to an embodiment.
Figs. 16A-16D illustrate an ablation probe in first, second, third, and fourth positions, respectively, according to another embodiment.
Fig. 17 is a block diagram of a computer according to an embodiment.

### Detailed Description

An ablation probe includes a proximal electrical sensor, a distal electrical sensor, and an ablation electrode. The ablation electrode is located between the proximal and distal electrical sensors. The ablation probe is configured to be inserted into a mammalian subject (e.g., a human), for example to locate and damage a target neural ganglion (e.g., the DRG). The electrical sensors are configured to measure an electrical characteristic (e.g., resistance or impedance) as the ablation probe is inserted into the subject. The electrical characteristic measured by an electrical sensor changes when that electrical sensor comes into physical contact with the target neural ganglion. The electrical characteristic measured by the electrical sensors is used to determine when the injection port is located within and/or aligned with the target neural ganglion.

In one embodiment, the ablation probe is configured so that both electrical sensors can simultaneously physically contact the target neural ganglion. In this embodiment, the magnitude of the electrical characteristic measured by both electrical sensors increases (or alternately decreases) compared to a baseline value or magnitude when the electrical sensors are in physical contact with the target neural ganglion, which indicates that the ablation probe is located within and/or aligned with the target neural ganglion.

In another embodiment, the ablation probe is configured so that only one electrical sensor can physically contact the target neural ganglion at any one time. In this embodiment, the magnitude of the electrical characteristic measured by the distal electrical sensor increases (or alternately decreases) compared to a baseline value or magnitude when the distal electrical sensor has physically contacted the target neural ganglion while the magnitude of the electrical characteristic measured by the proximal electrical sensor remains about equal to the baseline magnitude. As the ablation probe is inserted further distally, the magnitude of the electrical characteristic measured by the distal electrical sensor returns to about the baseline magnitude and the magnitude of the electrical characteristic measured by the proximal electrical sensor remains about equal to the baseline magnitude, which indicates that the target neural ganglion is located between the distal and proximal electrical sensors and that the ablation electrode is located within and/or aligned with the target neural ganglion.

The distal and proximal electrical sensors are electrically coupled to a power source/supply, which can be internal or external to the ablation probe. In addition, the distal and proximal electrical sensors or their wires/leads can be electrically coupled to a computer that can be programmed to monitor the electrical characteristic measured by the distal and proximal electrical sensors and produce an output control signal when the ablation electrode is located within and/or aligned with the target neural ganglion. An interlock switch can be electrically connected in series with the power source/supply and the ablation electrode. The interlock switch can be configured to have a default open state in which the power source/supply is electrically disconnected from the ablation electrode. The interlock switch can transition from the default open state to a closed state when the ablation electrode is located within and/or aligned with the target neural ganglion (e.g., in response to the output control signal produced by the computer). When the interlock switch is in the closed state, the power source/supply is electrically connected to the ablation electrode.

In some embodiments, the ablation probe can include multiple ablation electrodes and/or more than two electrical sensors.

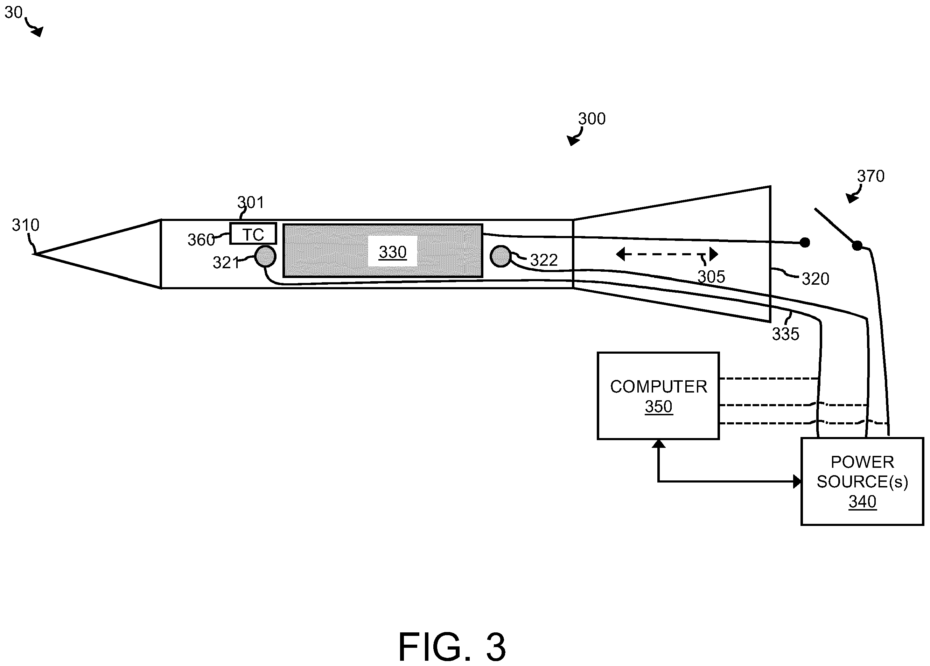

**Fig. 3** is a simplified view of an ablation system 30 according to an embodiment. The system 30 includes an ablation probe 300 having an elongated body 301, a tapered distal end 310, and a proximal end 320. The tapered distal end 310 is configured to be inserted through an opening or orifice in a subject. For example, the distal end 310 can be inserted through a surgical opening or transcutaneous puncture in a mammalian subject's (e.g., a human's) back near the DRG. The tapered distal end 310 can terminate in a point, a planar surface, a rounded surface, or another termination. The width of the termination of the tapered distal end 310 is preferably smaller than the width of the elongated body 301. The proximal end 320 can be flared or it can have the same width as the elongated body 301.

The overall length of the probe 300 can be about 10 cm to about 15 cm, including about 11 cm, about 12 cm, about 13 cm, about 14 cm, and/or any value or range between any two of the foregoing lengths. In other embodiments, the overall length of the probe 300 can be less than about 10 cm (e.g., about 7.5 cm, about 5 cm, or another length) or greater than about 15 cm (e.g., about 17.5 cm, about 20 cm, or another length). The overall length of the probe 300 is measured along or parallel to longitudinal axis 305 and from distal to proximal ends 310, 320. As used herein, "about" means plus or minus 10% of the relevant value.

The elongated body 301 includes distal and proximal electrical sensors 321, 322 and an electrode 330. The electrode 330 is located between the electrical sensors 321, 322 along the length of the elongated body 301 (e.g., along or parallel to a longitudinal axis 305 that extends from the distal end 310 to the proximal end 320). The electrical sensors 321, 322 and the electrode 330 comprise a conductive material that is preferably biocompatible. For example, the electrical sensors 321, 322 and the electrode 330 can comprise a biocompatible conductive metal such as platinum or a biocompatible conductive polymer such as a polythiophene (e.g., poly(3,4-ethylenedioxythiophene) (PEDOT)). Additionally, the probe can be constructed using thin-film and/or other deposition techniques. The electrode 330 and the electrical sensors 321, 322 are preferably aligned with respect to axis 305.

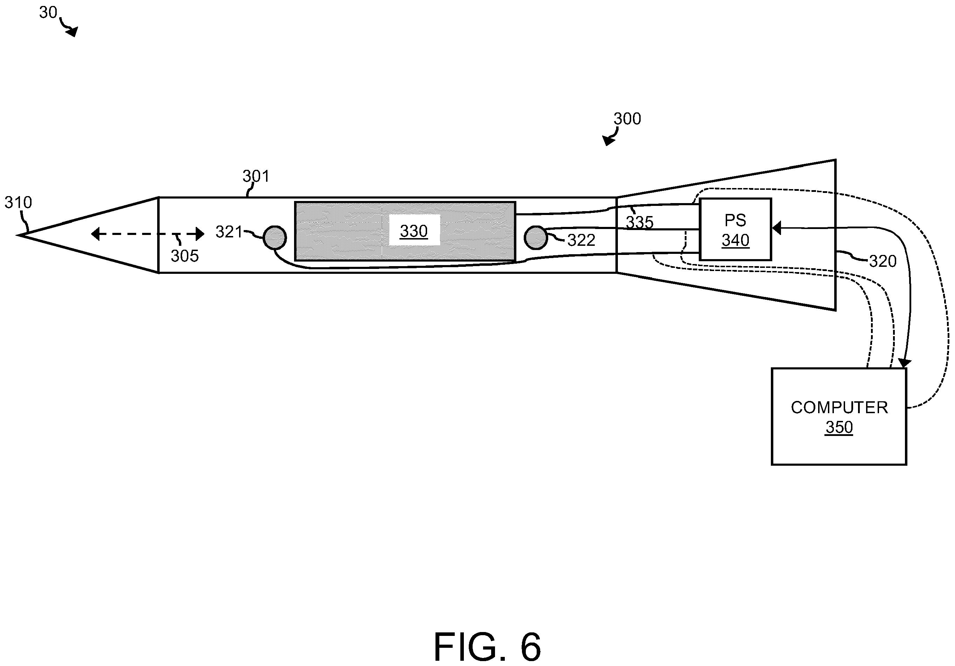

The electrical sensors 321, 322 and the electrode 330 are electrically coupled to one or more power sources 340 via respective wires or leads 335 that extend through the proximal end 320 of the probe 30. The power source(s) 340 can apply an AC or DC voltage across the positive and negative terminals of each electrical sensor 321, 322. The power source(s) 340 can be one or more external power sources or supplies, as illustrated in Fig. 3 or one or more internal power sources or supplies, as illustrated in **Fig. 6****.** When the power source(s) 340 includes one or more internal power sources, the internal power source(s) can comprise one or more batteries and/or other internal power supply(ies) or source(s). It is noted that the embodiment illustrated in Fig. 6 can include interlock switch 370, which is not illustrated therein for clarity purposes only.

The electrical characteristics measured by the electrical sensors 321, 322 can change when the electrical sensors 321, 322 are electrically coupled to different materials. For example, the effective resistance or impedance across the positive and negative terminals of each electrical sensor 321, 322 can vary as the electrical sensors 321, 322 are inserted into the surgical site and placed in physical contact with different anatomical features and substances. The effective resistance or impedance across the positive and negative terminals of the electrical sensor 321, 322 can be relatively low when the electrical sensor 321, 322 is in physical contact with a bodily fluid (e.g., blood) and can be relatively high when the electrical sensor 321, 322 is in physical contact a nerve such as the DRG. Alternatively, the effective resistance or impedance across the positive and negative terminals of the electrical sensor 321, 322 can be relatively high when the electrical sensor 321, 322 is in physical contact with a bodily fluid (e.g., blood) and can be relatively low when the electrical sensor 321, 322 is in physical contact a nerve such as the DRG. Thus, the electrical characteristics measured by the electrical sensors 321, 322 can change when one or both electrical sensors 321, 322 are in physical contact with the DRG, which can indicate when the injection port 330 is positioned in the DRG.

A computer or controller 350 can be electrically coupled to the wires or leads 335 for the sensors 321, 322 to determine one or more electrical characteristics (e.g., voltage, resistance, impedance, and/or another electrical characteristic) measured by the electrical sensors 321, 322. Additional electrical sensors can be included in other embodiments. Additionally or alternatively, the electrical sensors 321, 322 can be in wireless communication with the computer 350. For example, the electrical sensors 321, 322 and computer 350 can each include a short-range wireless transceiver and antenna that can be compatible and/or support Bluetooth.

The computer/controller 350 can be electrically coupled to the wires or leads 335 for the electrode 330 and/or to a current or voltage sensor that is coupled to the wires/leads 335 for the electrode 330. The computer/controller 350 can also be in electrical communication with and/or electrically coupled to the power source(s) 340 to control the voltage supplied to the electrical sensors 321, 322, to control the power supplied to the electrode 330, and/or to determine the electrical characteristic(s) measured by the electrical sensors 321, 322.

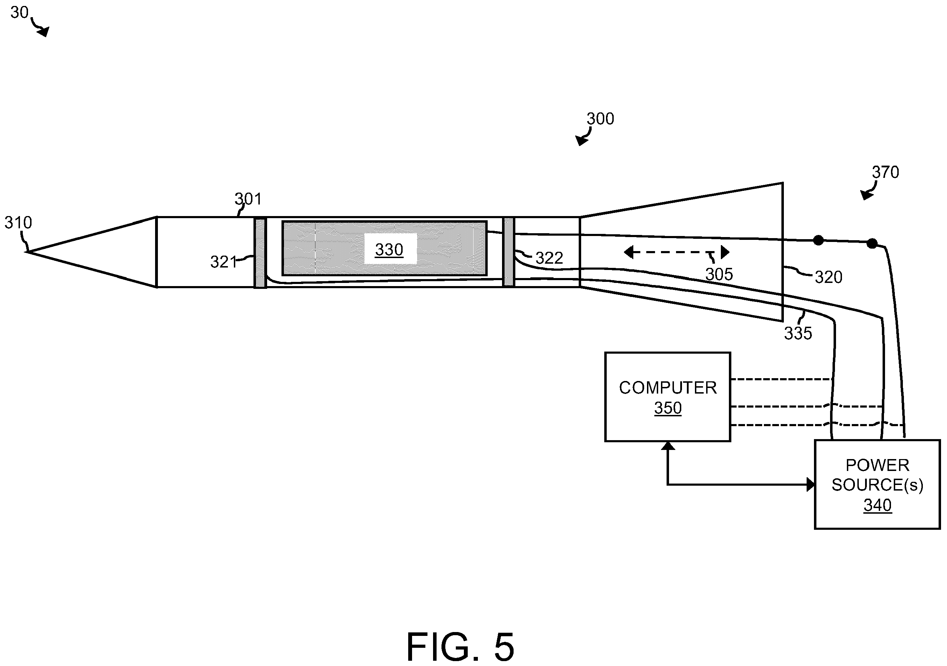

The electrical sensors 321, 322 can measure one or more electrical characteristics of the anatomical features of the subject as the probe 300 is inserted into the surgical site. The electrical characteristic(s) measured by the electrical sensors 321, 322 can change when one or both electrical sensors 321, 322 are in physical contact with the DRG, which can indicate when the electrode 330 is positioned in and/or in physical contact with the DRG. Electrical sensors 321, 322 may also measure biopotential that are evoke by stimulation (physical touch and/or stimulation evoked) of the sensory receptors. The electrical sensors 321, 322 can be circular, rectangular, square, or another shape. The electrical sensors 321, 322 are preferably identical in size and shape, but can have different sizes and/or shapes in other embodiments. In some embodiments, one or both electrical sensors 321, 322 can be or include band or ring contacts that extend along some or all of the perimeter of the elongated body 301 (e.g., as illustrated in **Fig. 5****).** In other embodiments, the electrical sensors 321, 322 can include both band/ring contacts and circular (or other shape) contacts, for example a hybrid of Figs. 3 and 5.

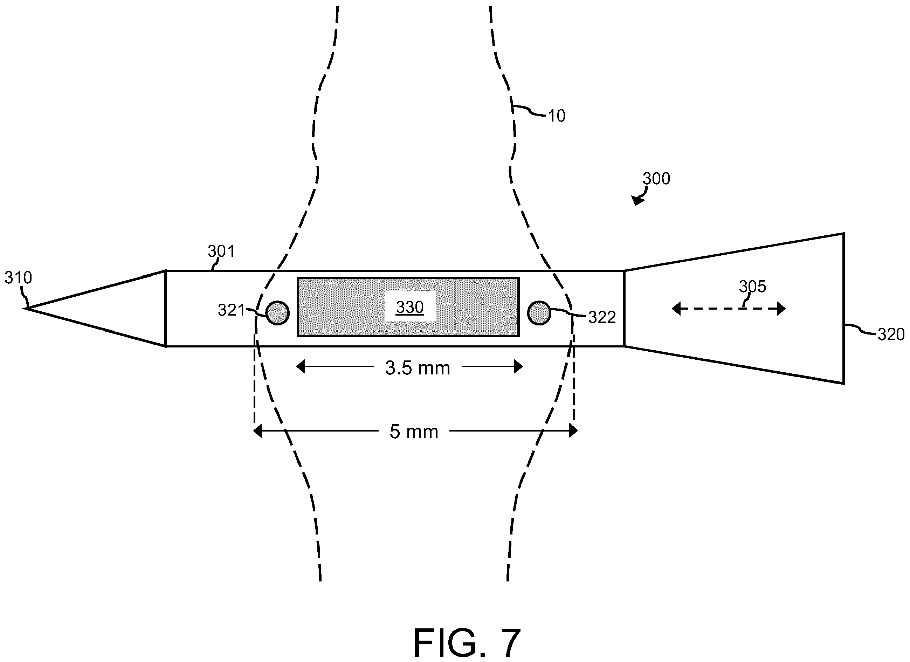

In one embodiment, the electrical sensors 321, 322 are spaced apart along the longitudinal axis 305 such that both electrical sensors 321, 322 can be in physical contact simultaneously with the DRG 10, for example as illustrated in **Fig. 7****.** In this embodiment, the distance along the longitudinal axis 305 between the electrical sensors 321, 322 is less than the thickness of the DRG as measured along the longitudinal axis 305. The wires or leads 335, power source(s) 340, and computer 350 are not illustrated in Fig. 7 for clarity purposes.

The electrical sensors 321, 322 can be micro-contacts or micro-sensors. In an embodiment, each electrical sensor 321, 322 can have a surface area of about 20 microns² to about 14 mm², including about 40 microns², about 60 microns², about 80 microns², about 100 microns², about 500 microns², about 1 mm², about 5 mm², about 10 mm², or any value or range between any two of the foregoing surface areas [In other embodiments, the surface area of each electrical sensor 321, 322 can be less than about 20 microns² (e.g., between 10 microns² and 20 microns²) or greater than about 14 mm² (e.g., between about 14 mm² and about 15 mm²). The electrical sensors 321, 322 are preferably the same size but can be different sizes.

When the electrode 330 is positioned in and/or in physical contact the DRG, electric power can be applied to the electrode 330 to therapeutically treat the DRG. For example, the electric power can cause the electrode 330 to ablate, damage, and/or disable at least a portion (e.g., some or all) of the DRG. For example, the electrode 330 can provide about 20 Volts to about 38 Volts, including about 25 Volts, about 30 Volts, about 32 Volts, about 34 Volts, about 36 Volts, or any value or range between any two of the foregoing voltages. Additionally or alternatively, the electrode 330 can provide about 100 mA to about 147 mA or current, including about 105 mA, about 110 mA, about 115 mA, about 120 mA, about 125 mA, about 130 mA, about 135 mA, about 140 mA, about 145 mA, or any value or range between any two of the foregoing currents. The electrode 330 can be a mono-polar, a bi-polar, or a tri-polar electrode. The electric power applied to the electrode 330 can heat the DRG to about 75°C to about 90°C, including about 80°C, about 85°C, or any value or range between any two of the foregoing temperatures. The DRG can be heated to about 75°C to about 90°C for about one minute.

Ablating, damaging, and/or disabling the DRG can prevent the DRG from transmitting sensory signals to the brain or can limit the magnitude of the sensory signals transmitted to the brain (e.g., to prevent or limit the transmission of pain or other negative sensory signals to the brain). The electrical sensors (321 and 322) can be used before ablation of the tissue to ensure that the therapy is applied to the proper DRG, that is the DRG involved in the pain sensation, and after ablation to ensure that the ablation has been completed. The electrode 330 can have a length of about 3 mm to about 6 mm, including about 3.5 mm, about 4 mm, about 4.5 mm, about 5 mm, about 5.5 mm, or any value or range between any two of the foregoing lengths. In other embodiments, the length of the electrode 330 can be less than about 3 mm (e.g., between about 2 mm and about 3 mm) or greater than 6 mm (e.g., between about 6 mm and about 8 mm). The length of the electrode 330 is measured along or parallel to the longitudinal axis 305. The "width" of the electrode 330 extends along some or all of the perimeter (e.g., circumference) of the elongated body 301. In one embodiment, the electrode 330 extends around the circumference of the elongated body 301 to form a ring, an annulus, or another shape. In another embodiment, the "width" of the electrode 330 extends along half, a quarter, or another fraction of the circumference of the elongated body 301. The diameter of the electrode can range from 0.7 to 1.6 mm which can be selected as a function on the depth and diameter of the targeted DRG. For long trajectories, a large diameter may be selected to provide the mechanical stability to aid in placement; whereas a small diameter electrode would be selected in the case of a small-diameter DRG.

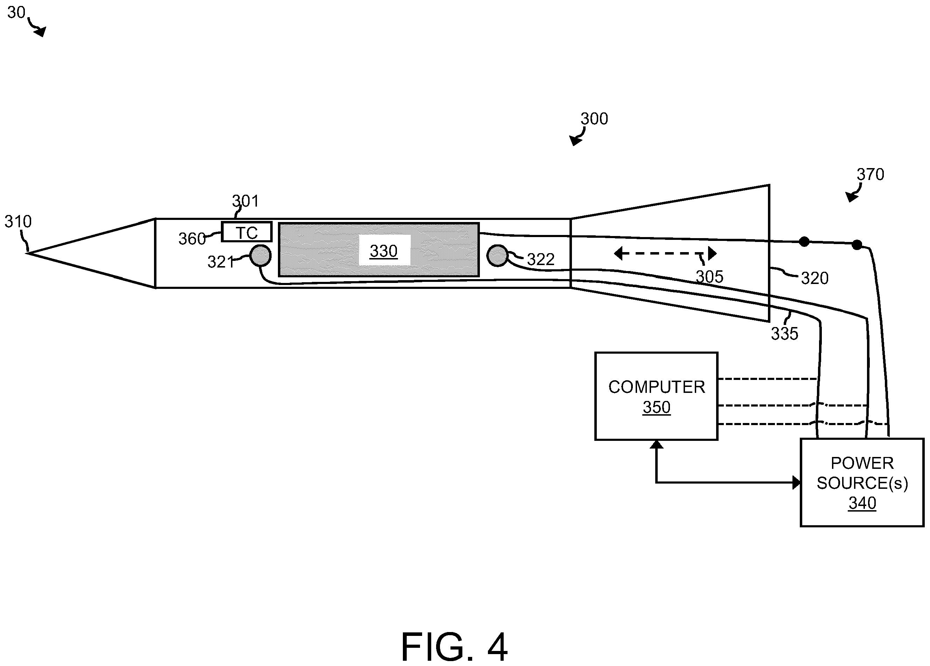

In some embodiments, an interlock switch 370 or another an electrical interlock can be electrically connected in series with the wire/lead 335 for the electrode 330. The switch 370 can have a default open state, as illustrated in Fig. 3, where the electrode 330 is electrically disconnected or electrically decoupled from its power supply 340 to prevent power from flowing into the electrode 330. The switch 370 can be in the default open state during insertion of the probe into the patient. The switch 370 can transition from the default open state to a closed state, as illustrated in **Fig. 4****,** in response to a voltage or current that can be supplied by the computer 350 or from the power source(s) in response to a control signal from the computer 350. The computer 350 can be configured to transition the switch 370 from the default open state to the closed state when the electrical characteristic(s) measured by the electrical sensors 321, 322 indicate that one or both electrical sensors 321, 322 is/are in physical contact with the DRG.

The size (e.g., length and width) of the electrode 330 can be selected according to the target DRG level (e.g., level C1-C8, L1-L5, S1-S4, or another level), which itself can have a variable size between subjects. For example, when a dimension of the target DRG 10 level is about 5.5 mm, the electrode 330 can be selected to have a length that is less than or equal to about 5.5 mm, such as about 3.5 mm, as illustrated in the example dimensions in Fig. 7.

The probe 300 can optionally include one or more thermocouples 360 (Fig. 3), located near (e.g., proximal to, next to and/or adjacent to) the lesioning contact on the electrode 330 (e.g., within about 2 mm of the electrode 330) or at the external connection of the lesion probe, which can be used to measure the temperature of the DRG 10 during therapeutic treatment with the electrode 330. The temperature can be used to confirm that the electric power has ablated, damaged, and/or disabled the DRG 10. Temperature for ablation can range from about 80°C to about 90°C for about 1 minute and can be repeated for incomplete ablation. Incomplete ablations can be determined by examination of the signal on electrical sensors 321 and 322.

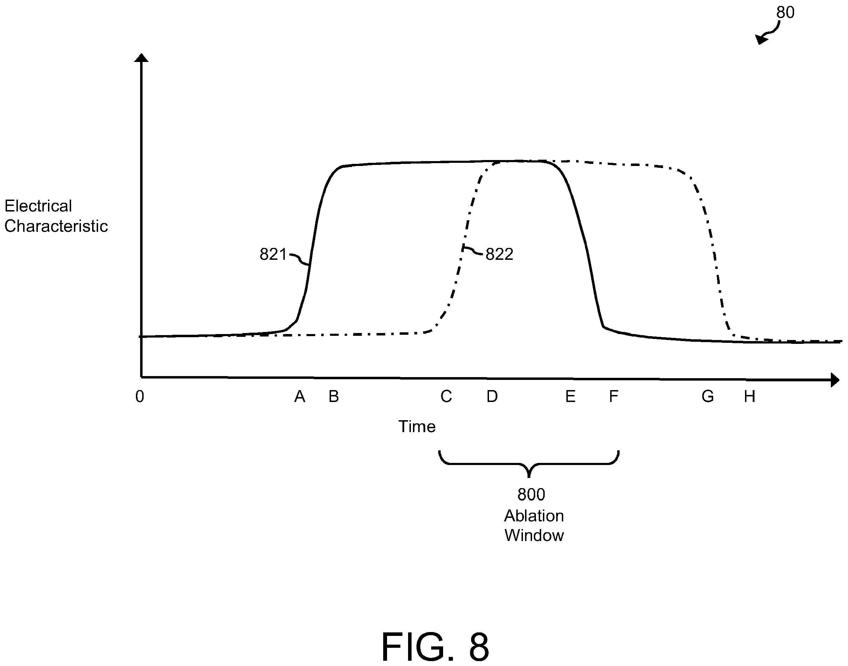

**Fig. 8** is an example graph 80 of a measured electrical characteristic versus time of the electrical sensors 321, 322 according to the embodiment illustrated in Fig. 7. Plot line 821 is the measured electrical characteristic of the distal electrical sensor 321 with respect to time as the probe 300 is inserted at a constant rate through a surgical site towards the DRG. Plot line 822 is the measured electrical characteristic of the proximal electrical sensor 322 with respect to time. At time 0, plot lines 821, 822 are at a baseline value which indicates that neither electrical sensor 321, 322 is in full or partial physical contact with the DRG 10. As can be seen, plot line 821 increases at time A, which corresponds to the start of physical contact between a portion of distal electrical sensor 321 and a portion of the DRG 10. Plot line 821 has an increased value and is at a plateau at time B, which indicates that the distal electrical sensor 321 is in full physical contact with the DRG 10.

Plot line 822 increases at time C (after time A), which corresponds to the start of physical contact between a portion of proximal electrical sensor 322 and a portion of the DRG 10. Plot line 822 has an increased value and is at a plateau at time D, which indicates that the proximal electrical sensor 322 is in full physical contact with the DRG 10. From time D to time E, both plot lines remain at the plateau indicating that both electrical sensors 321, 322 are in full physical contact with the DRG 10 over this time period.

Plot line 821 decreases at time E, which indicates that a portion of distal electrical sensor 321 is not in physical contact with the DRG 10. At time F, plot line 821 has returned to the baseline value which indicates that the distal electrical sensor 321 is not in partial or full contact with the DRG 10. In other words, the distal electrical sensor 321 has passed through the DRG 10 at time F. Plot line 822 decreases at time G, which indicates that a portion of proximal electrical sensor 322 is not in physical contact with the DRG 10. At time H, plot line 822 has returned to the baseline value which indicates that the proximal electrical sensor 322 is not in partial or full contact with the DRG 10. The proximal electrical sensor 322 has passed through the DRG 10 at time H.

It is noted that although graph 80 indicates that the measured electrical characteristic increases when an electrical sensor is physical contact with the DRG, in other embodiments, the measured electrical characteristic can decrease when the electrical sensor is physical contact with the DRG. The electrical characteristic can comprise electrical voltage, electrical resistance, electrical conductivity, electrical impedance, and/or another electrical characteristic.

The time interval from time C to time F in graph 80 corresponds to the time interval when it can be determined from the plot lines 821, 822 that the electrode 330, in the embodiment illustrated in Fig. 7, is located in the DRG 10. During this time period, the electrical characteristics indicate that one of the electrical sensors is in at least partial physical contact with the DRG and the other electrical sensor is in full physical contact with the DRG. Since the electrode 330 is located between the electrical sensors 321, 322, it can be assured that the electrode 330 is disposed in the DRG (e.g., in physical contact with the DRG) during this time interval. This time interval can be referred to as an ablation window 800.

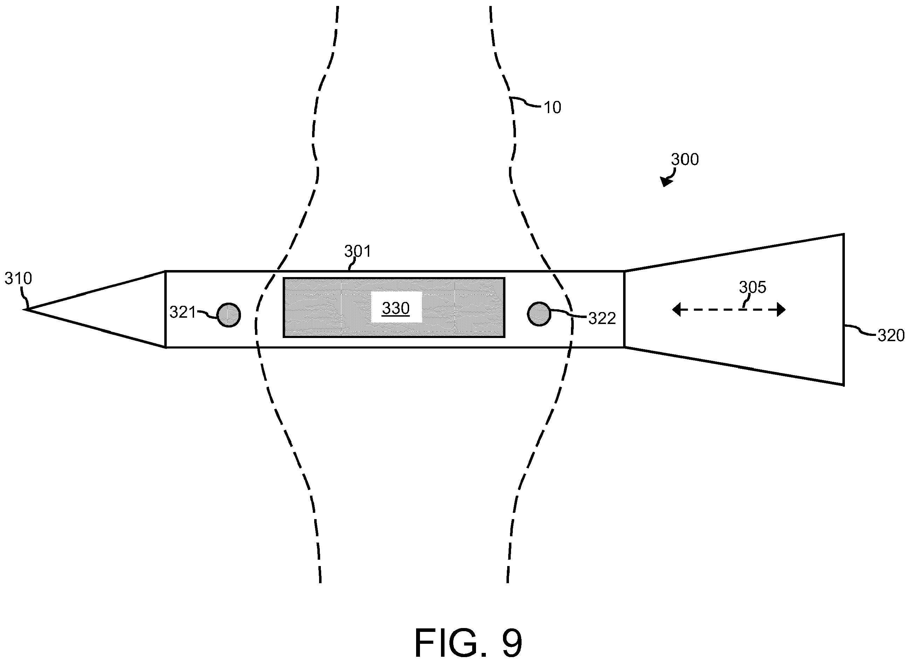

In another embodiment, the electrical sensors 321, 322 are spaced further apart from each other along the longitudinal axis 305 such that only one electrical sensor 321, 322 can be in physical contact with the DRG at any given time, for example as illustrated in **Fig. 9****.** In this embodiment, the distance along the longitudinal axis 305 between the electrical sensors 321, 322 is greater than the thickness of the DRG 10 as measured along the longitudinal axis 305. The wires or leads 330, power source(s) 340, and computer 350 are not illustrated in Fig. 9 for clarity purposes.

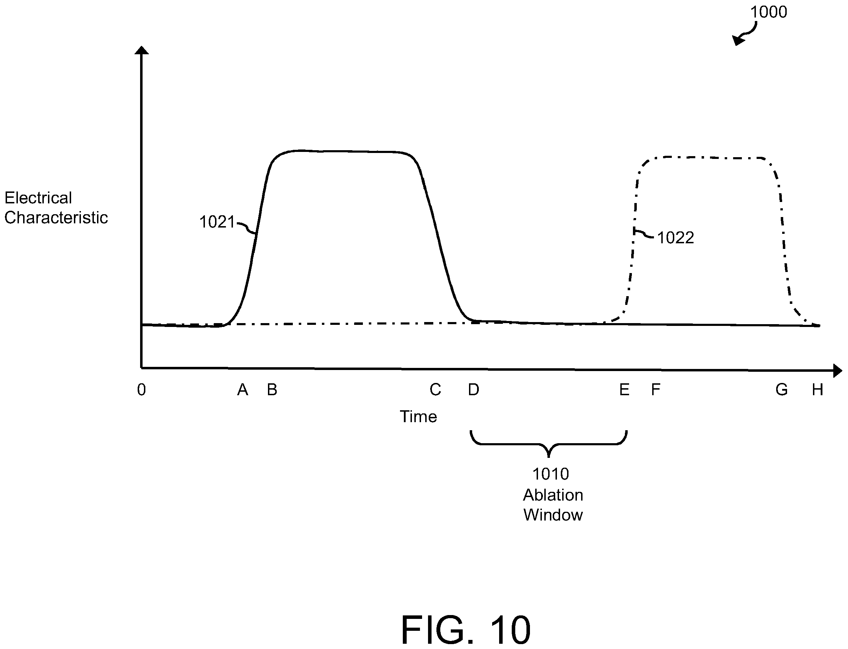

**Fig. 10** is an example graph 1000 of a measured electrical characteristic versus time of the electrical sensors 321, 322 according to the embodiment illustrated in Fig. 6. Plot line 1021 is the measured electrical characteristic of the distal electrical sensor 321 with respect to time as the probe 300 is inserted at a constant rate through a surgical site towards the DRG. Plot line 1022 is the measured electrical characteristic of the proximal electrical sensor 322 with respect to time. At time 0, plot lines 1021, 1022 are at a baseline value which indicates that neither electrical sensor 321, 322 is in full or partial physical contact with the DRG 10. As can be seen, plot line 1021 increases at time A, which corresponds to the start of physical contact between a portion of distal electrical sensor 321 and a portion of the DRG 10. Plot line 1021 has an increased value and is at a plateau from time B to time C, which indicates that the distal electrical sensor 321 is in full physical contact with the DRG 10 during this time interval. At time C, the plot line 1021 decreases, which indicates that a portion of distal electrical sensor 321 is not in physical contact with the DRG 10. At time D, plot line 1021 returns to the baseline value which indicates that the distal electrical sensor 321 is not in full or partial physical contact with the DRG 10. Thus, at time D the distal electrical sensor 321 has fully passed through the DRG 10.

Plot line 1022 remains at the baseline value from time 0 until time E when plot line 1022 increases, which corresponds to the start of physical contact between a portion of proximal electrical sensor 321 and a portion of the DRG 10. The time interval between time D and time E corresponds to the time when the distal electrical sensor 321 has passed through the DRG 10 and the proximal electrical sensor 322 has not physically contacted the DRG 10. Thus, the distal electrical sensor 321 is on the distal side of the DRG 10 and the proximal electrical sensor 322 is on the proximal side of the DRG during this time interval.

From time F to time G, plot line 1022 has an increased value and is at a plateau, which indicates that the proximal electrical sensor 322 is in full physical contact with the DRG 10 during this time interval. At time G, the plot line 1022 decreases, which indicates that a portion of proximal electrical sensor 322 is not in physical contact with the DRG 10. At time H, plot line 1022 returns to the baseline value which indicates that the proximal electrical sensor 322 is not in full or partial physical contact with the DRG 10. Thus, at time H the proximal electrical sensor 322 has fully passed through the DRG 10.

It is noted that although graph 1000 indicates that the measured electrical characteristic increases when an electrical sensor is physical contact with the DRG, in other embodiments, the measured electrical characteristic can decrease when the electrical sensor is physical contact with the DRG. The electrical characteristic can comprise electrical resistance, electrical conductivity, electrical impedance, or another electrical characteristic.

The time interval from time D to time E in graph 1000 corresponds to the time interval when it can be determined from the plot lines 1021, 1022 that the ablation contact 330, in the embodiment illustrated in Fig. 7, is located in the DRG. During this time period, the electrical characteristics indicate that neither electrical sensor is in at least partial physical contact with the DRG. Since the ablation contact 330 is located between the electrical sensors 321, 322, it can be assured that the ablation contact is disposed in the DRG during this time interval. This time interval can be referred to as an ablation window 1010.

In either embodiment, computer 350 can be programmed to generate a signal, such as an audible or visual signal, to indicate when the probe 300 is positioned in the ablation window 1010 such that the ablation contact 330 is disposed in the DRG.

In addition to the above, the electrical sensors 321, 322 can measure or detect a sensory signal in the DRG which can be used to confirm that one or both electrical sensors 321, 322 are positioned in the DRG and not in another anatomical feature. When the probe 300 is positioned in an anatomical feature that the operator believes is the DRG, the operator (or another person) can produce a sensory signal by mechanically stimulating (e.g., by touching or stroking) and/or electrically stimulating (e.g., using an electrode) the shin or fingers of the subject, which can cause electrical signals to pass through the sensory nerves in the DRG. If one or both electrical sensors 321, 322 detects these electrical signals, it can be determined that the electrical sensor(s) is/are located in the DRG. If neither electrical sensor detects the electrical signals, then it can be determined that the electrical sensors are not located in the DRG.

In some situations, more than one DRG can be connected to the anatomical feature where the subject feels pain (or other undesired sensory signal). In these situations, damaging/disabling one DRG may not remove (or fully remove) the subject's pain symptoms.

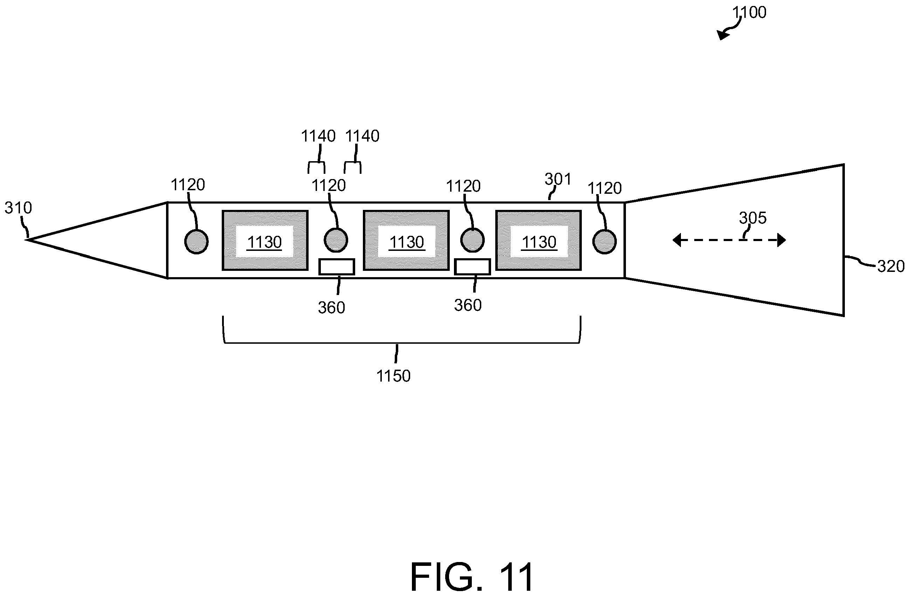

**Fig. 11** is a simplified view of a multi-electrode ablation probe 1100 according to another embodiment. Probe 1100 is the same as probe 300 except that probe 1100 includes multiple electrodes 1130 and/or three or more electrical sensors 1120. Each electrode 1130 can be the same as or different than electrode 330. Each electrical sensor 1120 can be the same as or different than electrical sensor 321, 322.

The overall length of the probe 1100 can be the same as or different than the overall length of probe 30. The overall length of probe 1100 is measured along or parallel to longitudinal axis 305 and from distal to proximal ends 310, 320.

In some embodiments, each electrode 1130 can have a smaller length, along or parallel to the longitudinal axis, compared to the electrode 330. For example, each electrode 1130 can have a length of about 1.75 mm to about 3 mm, including about 2.25 mm, about 2.5 mm, about 2.75 mm, and any value or range between any two of the foregoing lengths. In other embodiments, the length of each electrode 1130 can be less than about 1.75 mm (e.g., between about 1.5 mm and about 1.75 mm) or more than about 3 mm (e.g., between about 3 mm and about 3.5 mm). The electrodes 1130 preferably have the same length but at least one electrode 1130 can have a different length in other embodiments.

The "width" of each electrode 1130, as measured with respect to axis 305, can be the same as the "width" of electrode 330. The electrodes 1130 preferably have the same width but at least one electrode 1130 can have a different width in other embodiments.

The electrical sensors 1120 and electrodes 1130 have an alternating pattern in which one electrode 1130 is located between two electrical sensors 1120. The alternating pattern can include a different number (e.g., higher or lower number) of electrical sensors 1120 and electrodes 1130 than that illustrated in Fig. 11. The electrodes 1130 and the electrical sensors 1120 are preferably aligned with respect to axis 305.

Neighboring electrical sensors 1120 and electrodes 1130 are preferably spaced evenly along or parallel to the longitudinal axis 305. For example, a space 1140 between each neighboring electrical sensor 1120 and electrode 1130 (and between each neighboring electrode 1130 and electrical sensor 1120) is preferably the same along the alternating pattern. The space 1140 is measured along or along or parallel to the longitudinal axis 305. The space 1140 can be about 0.2 mm to about 0.3 mm including about 0.25 mm. When each electrode 1130 has a length of about 2.25 mm and the space 1140 is about 0.25 mm, the total length 1150 along the probe 1100 from the proximal end to the distal end of the electrodes 1130 is about 7.5 mm.

The electrical sensors 1120 and electrodes 1130 can be electrically coupled to the power source(s) 340 in the same manner that the electrical sensors 321, 322 and electrode 330 are electrically coupled to power source(s) 340 (e.g., using wires/leads 335). A computer or controller, such as computer 350, can be electrically coupled to the power source(s) 340 and/or to the wires/leads to the electrical sensors 1120 and electrodes 1130 in the same manner as in probe 300 (e.g., illustrated in Fig. 3). As such, system 30 can include probe 1100 instead of, or in addition to, probe 300.

The probe 1100 can optionally include one or more thermocouples 360, located near (e.g., proximal to, next to and/or adjacent to) one or more electrodes 1120 (e.g., within about 2 mm of the electrode 330). For example a thermocouple 360 can be located between a pair of neighboring/adjacent electrodes 1120. Alternatively, probe can include multiple thermocouples 360, where each thermocouple is located between a respective pair of neighboring electrodes 1120. The thermocouple 360 can additionally or alternatively be located at the external connection of the lesion probe.

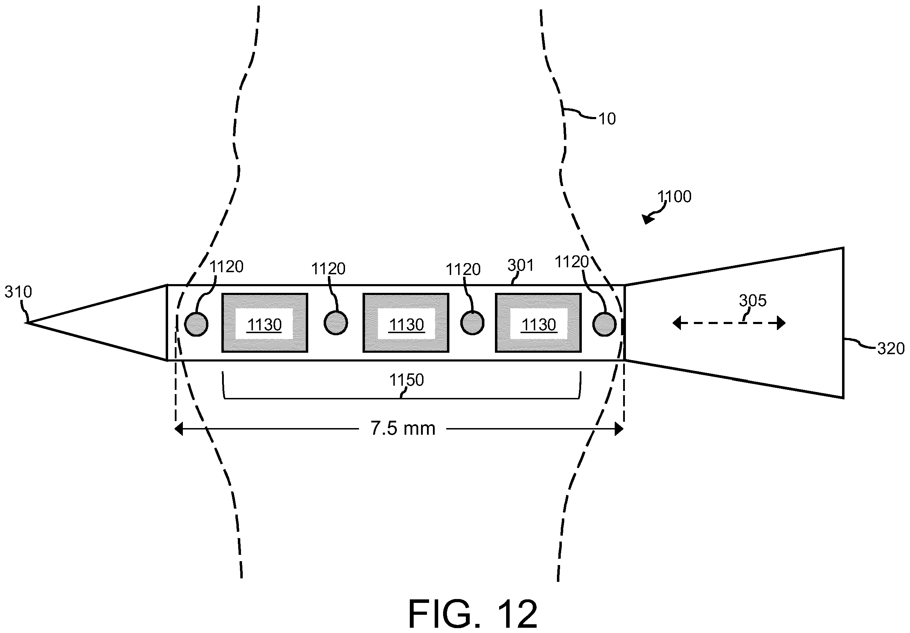

It may be advantageous to use multiple electrodes 1130 instead of a single electrode 330 when a dimension of the target DRG 10 level is long (e.g., greater than about 6 mm). For example, when a dimension of the target DRG 10 level is about 7.5 mm, the electrodes 1130 can be selected to have a total length 1150 that is about 5.5 mm to about 7.5 mm, including about 6 mm, about 6.5 mm, about 7 mm, and any value or range between any two of the foregoing lengths, as illustrated in **Fig. 12****.**

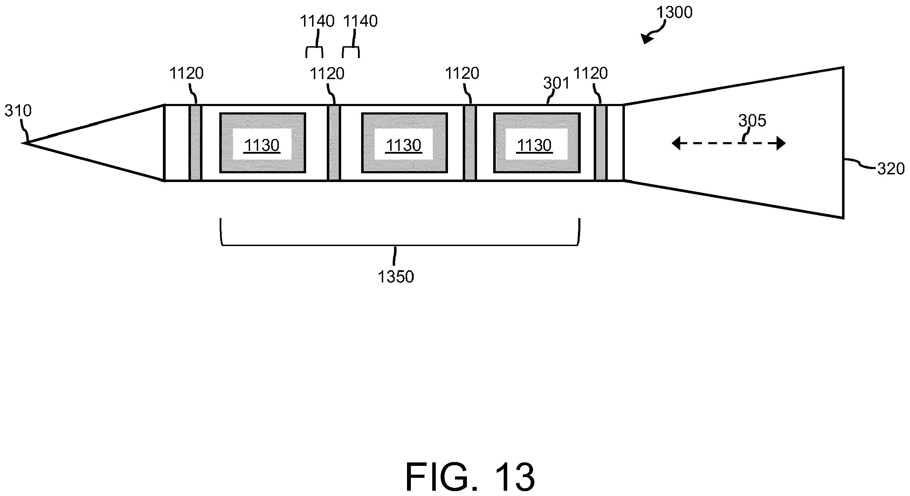

**Fig. 13** is a simplified view of a probe 1300 according to another embodiment. Probe 1300 is the same as probe 1100 except that in probe 1300 the electrical sensors 1120 are band contacts that extend along some or all of the perimeter (e.g., circumference) of the elongated body 301. When the bands contacts extend around all of the perimeter (e.g., circumference) of the elongated body 301, the band contacts can form ring contacts. Probe 1300 can include one or more optional thermocouples 360, which is/are not illustrated in this figure for clarity purposes only.

The overall length 1350 of the probe 1300 can be the same as or different than the of probe 300 and/or or probe 1100. The overall length 1350 of probe 1300 is measured along or parallel to longitudinal axis 305 and from distal to proximal ends 310, 320.

The bands or rings can have a width, measured along or parallel to the longitudinal axis 305, of about 0.4 mm to about 0.6 mm including about 0.5 mm. When each electrode 1130 has a length of about 2.25 mm, each band contact of electrical sensor 1120 has a width of about 0.5 mm, and the space 1140 is about 0.25 mm, the total length 1150 along the probe 1300 from the proximal end to the distal end of the electrodes 1130 is about 8.75 mm.

In other embodiments, the electrical sensors 1120 can include both band/ring contacts and circular (or other shape) contacts, for example a hybrid of Figs. 12 and 13.

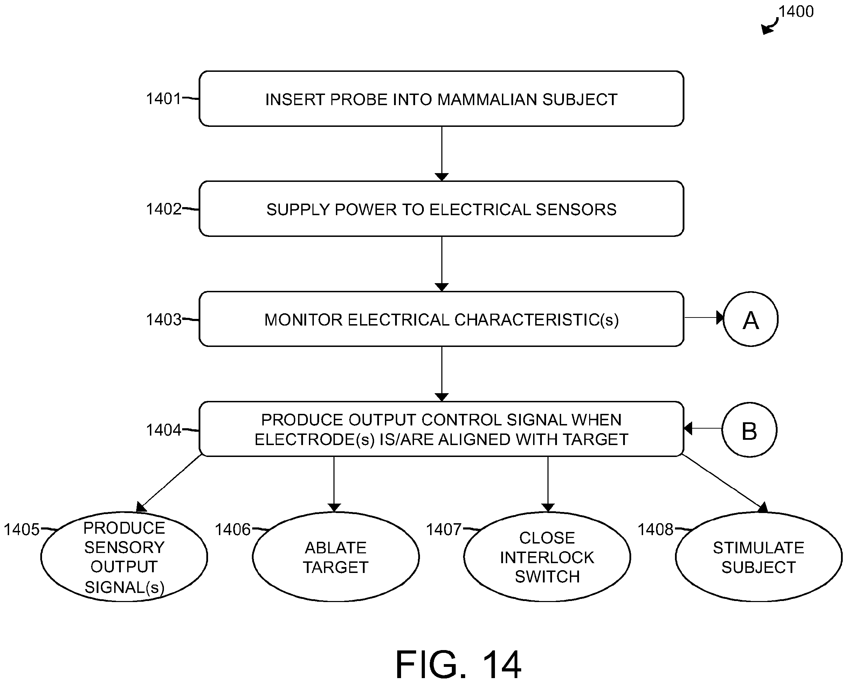

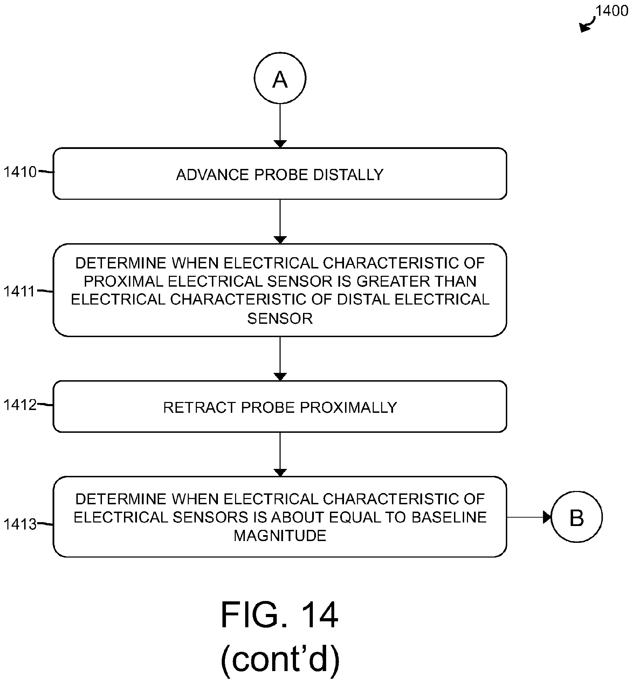

**Fig. 14** is a flow chart of a method 1400 for locating and damaging a target anatomical feature according to an embodiment. Method 1400 can be performed using system 30.

In step 1401, a probe is inserted into a mammalian subject in the region of the target anatomical feature, such as a neural ganglion (e.g., a DRG). The probe can be inserted into a surgical site or into a natural body orifice. The probe can be the same as probe 30, probe 1100, probe 1300, or another probe.

In step 1402, power (e.g., a voltage) is supplied to two or more electrical sensors on the probe. The power can be supplied from an internal or external power source, such as power source(s) 340. The power can be AC power or DC power, which can produce an AC voltage or a DC voltage, respectively, across the positive and negative electrical terminals of each electrical sensor 321, 322. The electrical sensors can be the same as electrical sensors 321, 322, and/or 1120.

In step 1403, one or more electrical characteristics measured by the electrical sensors is/are monitored with a computer (e.g. computer 350) that has an input that is electrically coupled to the output of the electrical sensors. For example, the computer 350 can be electrically coupled to the wires or leads 345 that electrically couple the power source(s) 340 to the electrical sensors 321, 322, and/or 1120. Additionally or alternatively, the computer 350 can be in wireless communication with the electrical sensors 321, 322, and/or 1120.

Steps 1401-1403 can occur simultaneously such that the probe is inserted and being positioned while power is supplied to the electrical sensors and the measured electrical characteristic(s) is/are monitored.

In step 1404, the computer 350 produces an output control signal when the computer determines that the electrical characteristic(s) measured by the electrical sensors 321, 322, and/or 1120 correspond to an ablation window (e.g., ablation window 800 or ablation window 1010) in which one or more electrodes (e.g., electrodes 330, 1130) is/are aligned with and/or located in (in physical contact with) the target anatomical feature (e.g., a target neural ganglia such as a target DRG).

The output control signal can optionally include or produce one or more sensory output signals in step 1405 to alert a user or operator. For example, the output control signal can include an output audio signal, an output video/graphical signal, and/or an output haptic signal (e.g., a vibration). Additionally or alternatively, the output control signal can cause the power source(s) for the electrode(s) to automatically apply power to the electrode(s) to ablate the target anatomical feature in step 1406, which can damage one or more nerves in the target anatomical feature. The temperature of the target anatomical feature can optionally be monitored by the computer, using a thermocouple in electrical communication with the computer, during and/or after step 1406 to determine whether the measured temperature is higher than a predetermined ablation threshold that corresponds to damage/necrosis of the nerves of the target anatomical feature. The computer can cause the ablation to stop when the measured temperature is higher than the predetermined ablation threshold for a predetermined time period. The predetermined ablation threshold can be within a temperature range of about 80°C to about 90°C, including about 85°C or any value or range between any two of the foregoing temperatures. The predetermined time period can be 45 seconds to about 75 seconds, including about 50 seconds, about 55 seconds, about 60 seconds, about 65 seconds, about 70 seconds, any value or range between any two of the foregoing time periods. The computer can cause the ablation to stop by sending a control signal to the power source(s) that cause the power source to stop applying power to the electrode.

Additionally or alternatively, the output control signal can cause a safety interlock switch (e.g., interlock switch 370) to close in step 1407 to electrically couple the electrode(s) to the power supply(ies), which can allow power to be applied to the electrode(s) to ablate the target anatomical feature.

In some embodiments, the operator (or another person) can produce a sensory signal by mechanically stimulating (e.g., by touching or stroking) and/or electrically stimulating (e.g., using an electrode) the shin or fingers of the subject in step 1408, which can cause electrical signals to pass through the sensory nerves in the target anatomical feature. If one or more electrical sensors detect(s) these electrical signals, it can be determined that the electrical sensor(s) is/are located in (e.g., in physical contact with) the target anatomical feature. If neither electrical sensor detects the electrical signals, then it can be determined that the electrical sensors are not located in (e.g., in physical contact with) the target anatomical feature, in which case the probe can be repositioned and steps 1402-1404 and 1408 can be repeated to determine whether the electrical sensor(s) detects the electrical signals at the new position of the probe. Ablation (step 1406) can occur after it is determined that the electrical sensor(s) detects the electrical signals at the position (or new position) of the probe.

In some embodiments, a combination of steps 1405-1408 can occur after step 1404. For example, after the computer produces the output control signal (in step 1404) that indicates that the electrical characteristic(s) correspond to an ablation window, a sensory output signal can be produced in step 1405 and/or the safety interlock switch can be closed in step 1407. Additionally or alternatively, the mammalian subject can be manually stimulated in step 1408 to confirm that the probe is located in (e.g., in physical contact with) the target anatomical feature (where steps 1402-1404 and 1408 can be repeated for different probe positions when the probe is not initially located in the target anatomical feature). Ablation (step 1406) can occur after it is determined that the electrical sensor(s) detects the electrical signals at the position (or new position) of the probe.

In some embodiments, additional positioning of the probe may be required or desired to determine whether one or more electrical sensors is/are located in (e.g., in physical contact with) the target anatomical feature.

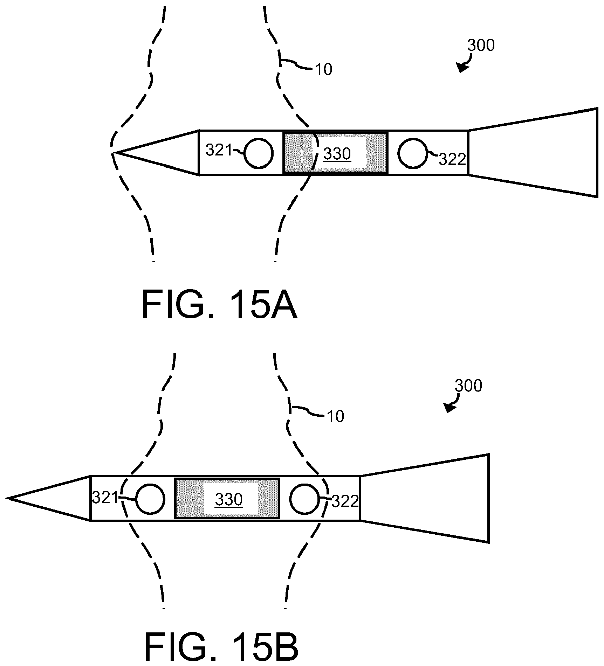

During steps 1401-1403 and when the probe has the configuration of Fig. 7, the probe is inserted to a first position where the distal electrical sensor 321 is in physical contact with the DRG 10 and the proximal electrical sensor 322 is not in physical contact with the DRG 10, as illustrated in **Fig. 15A****.** At the first position, the magnitude of the electrical characteristic measured by the distal electrical sensor 321 is higher than the magnitude of the electrical characteristic measured by the distal electrical sensor 322, such as between time B and time C in graph 80 (Fig. 8). The probe is inserted further to a second position that is distal to the first position, as illustrated in **Fig. 15B****.** At the second position, both electrical sensors 321, 322 are in physical contact with the DRG 10. At the second position, the magnitude of the electrical characteristic measured by the electrical sensors 321, 322 is high, which indicates that the probe is positioned in the ablation window 800 (Fig. 8).

When the probe has the configuration of Fig. 9, additional positioning may be desired after the output control signal is produced in step 1404 to confirm that the probe is positioned correctly with respect to the target neural ganglion. For example, referring to Fig. 10 it can be seen that the magnitude of the electrical characteristic during the ablation window 1010 (Fig. 10) is about the same as the baseline value or magnitude of the electrical characteristic from time 0 to time A. Therefore it may be desirable to confirm that a low-magnitude electrical characteristic signal measured by both electrical sensors corresponds to the ablation window 1010.

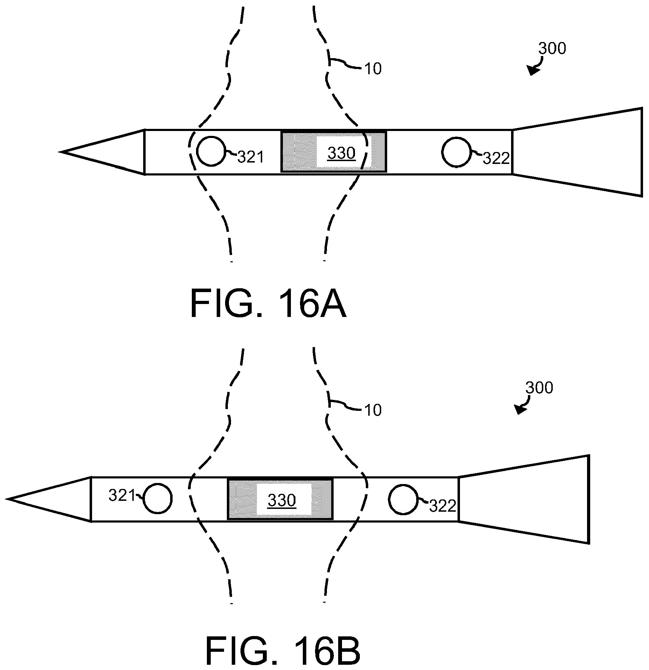

During steps 1001-1003, the probe of Fig. 9 is inserted to a first position where the distal electrical sensor 321 is in physical contact with the DRG 10 and the proximal electrical sensor 322 is not in physical contact with the DRG 10, as illustrated in **Fig. 16A****.** At the first position, the magnitude of the electrical characteristic measured by the distal electrical sensor 321 is higher than the magnitude of the electrical characteristic measured by the proximal electrical sensor 322, such as between time B and time C in graph 1000 (Fig. 10). The probe is inserted further to a second position that is distal to the first position, as illustrated in **Fig. 16B****.** At the second position, the DRG 10 is between the electrical sensors 321, 322 such that the electrical sensors 321, 322 are not in physical contact with the DRG 10. At the second position, the magnitude of the electrical characteristic measured by the electrical sensors 321, 322 is low and about equal to the baseline value or magnitude, such as in the ablation window 1010 of graph 1000.

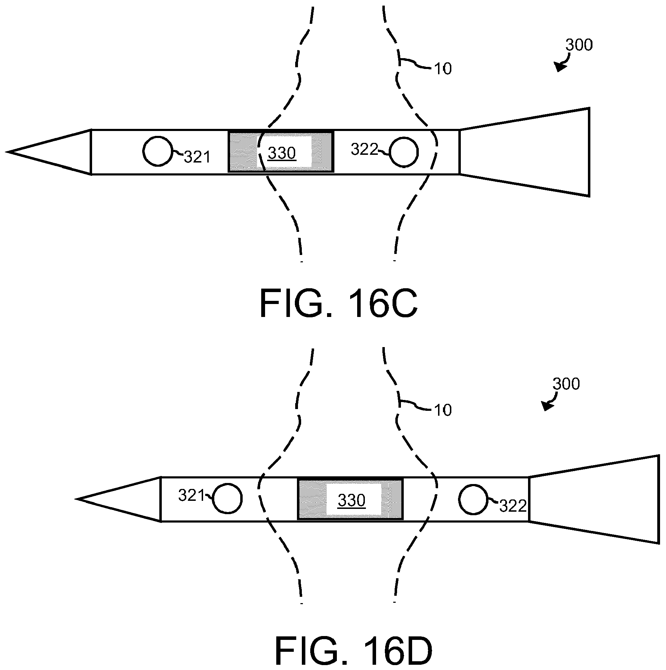

In optional step 1410 (via placeholder A), the probe can be advanced distally from the second position to a third position where the proximal electrical sensor 322 is in physical contact with the DRG 10 and the distal electrical sensor 321 is not in physical contact with the DRG 10, as illustrated in **Fig. 16C****.** At the third position, the magnitude of the electrical characteristic measured by the proximal electrical sensor 322 is higher than the magnitude of the electrical characteristic measured by the distal electrical sensor 321, such as between time F and time G in graph 1000.

In optional step 1411, the computer 350 can determine when the magnitude of the electrical characteristic measured by the proximal electrical sensor 322 is higher than the magnitude of the electrical characteristic measured by the distal electrical sensor 321. The computer 350 can produce an output signals, such as a sensory output signal (e.g., similar to the sensor output signal produced in step 1405), when the magnitude of the electrical characteristic measured by the proximal electrical sensor 322 is higher than the magnitude of the electrical characteristic measured by the distal electrical sensor 321, which can provide function as a confirmation signal for the operator.

In optional step 1412, the probe can be retracted proximally from the third position to a fourth position where the DRG 10 is between the electrical sensors 321, 322 such that the electrical sensors 321, 322 are not in physical contact with the DRG 10, as illustrated in **Fig. 16D****.** The fourth position is between the first and third positions and can be the same as the second position. At the fourth position, the magnitude of the electrical characteristic measured by the electrical sensors 321, 322 is low and about equal to the baseline value or magnitude, such as in the ablation window 1010 (Fig. 10). In optional step 1413, the computer 350 determines when the magnitude of the electrical characteristic measured by the electrical sensors 321, 322 is about the same and about equal to the baseline value or magnitude. After optional step 1413, the method 1400 returns to step 1404 (via placeholder B) to produce an output control signal. In some embodiments, for the probe of Fig. 9, the computer only produces the output control signal in step 1404 after the computer 350 sequentially determines (1) that the magnitude of the electrical characteristic measured by the proximal electrical sensor 322 is higher than the magnitude of the electrical characteristic measured by the distal electrical sensor 321 (step 1411) and then (2) that the magnitude of the electrical characteristic measured by the electrical sensors 321, 322 is about the same and about equal to the baseline value or magnitude (step 1413).

In addition to DRGs, the device and method can be used in other neural ganglia (e.g., facial ganglia, etc.), in brain targets (e.g., Parkinson's-knowing physiologically that you are in a specific location of the target, Epilepsy, etc.), and/or in other tissues of the body based on electrical properties (e.g., in a specific part of liver, in a vein and out or through the vein, subcutaneous -v- intramuscular, etc.).

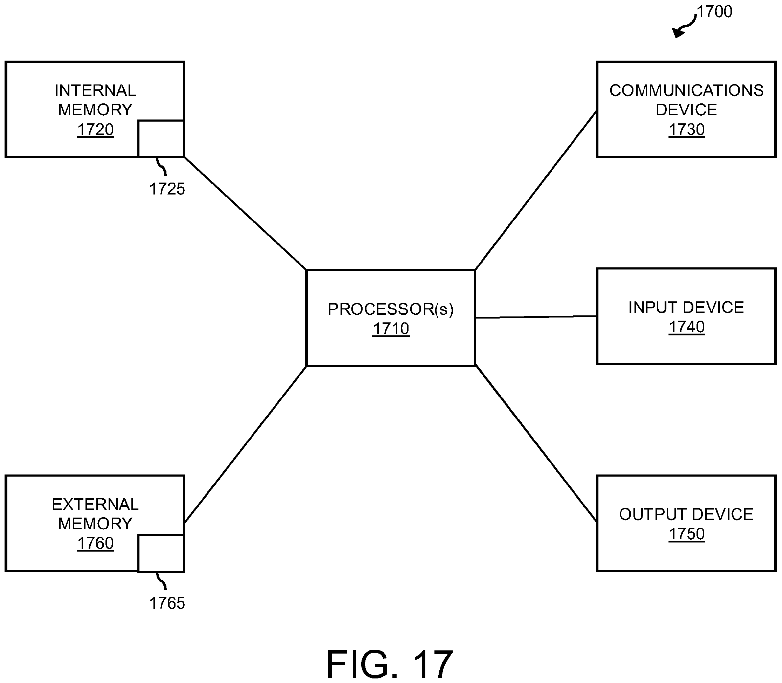

**Fig. 17** is a block diagram of a computer 1700 according to an embodiment. Computer 1700 can be the same as computer 350. The computer 1700 includes one or more microprocessors 1710. The microprocessor(s) 1710 can include one or more central processing units (CPUs), graphics processing units (GPUs), and/or other processors (e.g., hardware-based processors). The microprocessor(s) 1710 is/are in electrical communication with internal memory 1720, a communications device 1730, an input device 1740, and/or an output device 1750. The internal memory 1720 can include non-volatile computer memory that can store instructions 1725 (e.g., software, applications, computer programs) that can be executed and/or interpreted by the microprocessor(s) 1710 to perform one or more tasks, steps, and/or methods as described herein. The internal memory 1720 can also be used to store data for or relating to one or more tasks, steps, and/or methods as described herein. Additionally or alternatively, the external memory 1760 can store instructions 1765 (e.g., software, applications, computer programs) that can be executed and/or interpreted by the microprocessor(s) 1710 to perform one or more tasks, steps, and/or methods as described herein. The external memory 1760 can comprise one or more non-transitory computer-readable storage media

The communications device 1730 can include an input/output port, a modem, and/or a radio (e.g., for wireless communication). The communications device 1730 can allow the microprocessor(s) 1710 to send and/or receive signals and/or data. For example, the communications device 1730 can allow the microprocessor(s) 1710 to be in electrical communication with the electrical sensors on the probe, the power source(s), and/or the interlock switch.

The input device 1740 can include a keyboard, a mouse, a touchscreen, a microphone, and/or another input device. The output device 1750 can include a display screen, a speaker, a printer, and/or another output device.

The invention should not be considered limited to the particular embodiments described above. Various modifications, equivalent processes, as well as numerous structures to which the invention may be applicable, will be readily apparent to those skilled in the art to which the invention is directed upon review of this disclosure. The above-described embodiments may be implemented in numerous ways. One or more aspects and embodiments involving the performance of processes or methods may utilize program instructions executable by a device (e.g., a computer, a processor, or other device) to perform, or control performance of, the processes or methods.

In this respect, various inventive concepts may be embodied as a non-transitory computer readable storage medium (or multiple non-transitory computer readable storage media) (e.g., a computer memory of any suitable type including transitory or non-transitory digital storage units, circuit configurations in Field Programmable Gate Arrays or other semiconductor devices, or other tangible computer storage medium) encoded with one or more programs that, when executed on one or more computers or other processors, perform methods that implement one or more of the various embodiments described above. When implemented in software (e.g., as an app), the software code may be executed on any suitable processor or collection of processors, whether provided in a single computer or distributed among multiple computers.

Further, it should be appreciated that a computer may be embodied in any of a number of forms, such as a rack-mounted computer, a desktop computer, a laptop computer, or a tablet computer, as non-limiting examples. Additionally, a computer may be embedded in a device not generally regarded as a computer but with suitable processing capabilities, including a Personal Digital Assistant (PDA), a smartphone or any other suitable portable or fixed electronic device.

Also, a computer may have one or more communication devices, which may be used to interconnect the computer to one or more other devices and/or systems, such as, for example, one or more networks in any suitable form, including a local area network or a wide area network, such as an enterprise network, and intelligent network (IN) or the Internet. Such networks may be based on any suitable technology and may operate according to any suitable protocol and may include wireless networks or wired networks.

Also, a computer may have one or more input devices and/or one or more output devices. These devices can be used, among other things, to present a user interface. Examples of output devices that may be used to provide a user interface include printers or display screens for visual presentation of output and speakers or other sound generating devices for audible presentation of output. Examples of input devices that may be used for a user interface include keyboards, and pointing devices, such as mice, touch pads, and digitizing tablets. As another example, a computer may receive input information through speech recognition or in other audible formats.

The non-transitory computer readable medium or media may be transportable, such that the program or programs stored thereon may be loaded onto one or more different computers or other processors to implement various one or more of the aspects described above. In some embodiments, computer readable media may be non- transitory media.

The terms "program," "app," and "software" are used herein in a generic sense to refer to any type of computer code or set of computer-executable instructions that may be employed to program a computer or other processor to implement various aspects as described above. Additionally, it should be appreciated that, according to one aspect, one or more computer programs that when executed perform methods of this application need not reside on a single computer or processor but may be distributed in a modular fashion among a number of different computers or processors to implement various aspects of this application.

Computer-executable instructions may be in many forms, such as program modules, executed by one or more computers or other devices. Generally, program modules include routines, programs, objects, components, data structures, etc. that performs particular tasks or implement particular abstract data types. The functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

Thus, the disclosure and claims include new and novel improvements to existing methods and technologies, which were not previously known nor implemented to achieve the useful results described above. Users of the method and system will reap tangible benefits from the functions now made possible on account of the specific modifications described herein causing the effects in the system and its outputs to its users. It is expected that significantly improved operations can be achieved upon implementation of the claimed invention, using the technical components recited herein.

Also, as described, some aspects may be embodied as one or more methods. The acts performed as part of the method may be ordered in any suitable way. Accordingly, embodiments may be constructed in which acts are performed in an order different than illustrated, which may include performing some acts simultaneously, even though shown as sequential acts in illustrative embodiments.

## Claims

1. An ablation system comprising:
an ablation probe comprising:
an elongated body having a tapered distal end and a proximal end that are aligned along an axis;
first and second electrical sensors disposed on the elongated body; and
an electrode disposed on the elongated body between the first and second electrical sensors;
a power source electrically coupled to the first and second electrical sensors and to the electrode;
a computer having an input electrically coupled to the first and second electrical sensors to receive first and second output signals, respectively, from the first and second electrical sensors; and
a non-transitory computer-readable memory operatively coupled to the computer, the non-transitory computer-readable memory storing computer-readable instructions that, when executed by the computer, cause the computer to:
analyze an electrical characteristic measured by the first and second electrical sensors, and
produce an output control signal when the electrical characteristic measured by the first and second electrical sensors indicates that the electrode is aligned with a target anatomical feature.

2. The system of claim 1, further comprising an interlock switch electrically connected to the electrode, the power source, and the computer, wherein:
the interlock switch has a default open state in which the electrode is electrically disconnected from the power source and a closed state in which the electrode is electrically connected to the power source, the interlock switch configured to transition from the default open state to the closed state in response to the output control signal.

3. The system of claim 1, wherein the electrode is aligned with the target anatomical feature when the electrical characteristic measured by the first and second electrical sensors has about the same magnitude.

4. The system of claim 3, wherein the electrode is aligned with the target anatomical feature when the electrical characteristic measured by the first and second electrical sensors indicates that the first and second electrical sensors are in physical contact with the target anatomical feature.

5. The system of claim 4, wherein the electrode is aligned with the target anatomical feature when the magnitude of the electrical characteristic measured by the first and second electrical sensors is higher than a respective baseline magnitude.

6. The system of claim 1, wherein:
the first electrical sensor is located closer to the tapered distal end than the second electrical sensor, and
the electrode is aligned with the target anatomical feature when the electrical characteristic measured by the first electrical sensor indicates that the first electrical sensor has passed over the target anatomical feature and the electrical characteristic measured by the second electrical sensor indicates that the second electrical sensor is not in physical contact with the target anatomical feature.

7. The system of claim 1, wherein the electrical characteristic comprises a resistance or an impedance.

8. The system of claim 1, further comprising a thermocouple disposed on the elongated body adjacent to the electrode.

9. The system of claim 1, wherein the first and second electrical sensors comprise band contacts that extend along at least a portion of a perimeter of the elongated body.

10. The system of claim 9, wherein the band contacts comprise ring contacts that form a respective loop along the perimeter of the elongated body.

11. The system of claim 1, wherein the first and second electrical sensors and the electrode are aligned with respect to the axis.

12. A multi-electrode ablation system comprising:
a multi-electrode ablation probe comprising:
an elongated body having a tapered distal end and a proximal end that are aligned along an axis;
a plurality of electrical sensors disposed on the elongated body; and
a plurality of electrodes disposed on the elongated body, the electrodes and electrical sensors having an alternating arrangement in which each electrode is located between a respective neighboring pair of electrical sensors;
one or more power sources electrically coupled to the electrical sensors and the electrodes;
a computer having an input electrically coupled to the electrical sensors to receive respective output signals from the electrical sensors; and
a non-transitory computer-readable memory operatively coupled to the computer, the non-transitory computer-readable memory storing computer-readable instructions that, when executed by the computer, cause the computer to:
analyze an electrical characteristic measured by the electrical sensors, and
produce an output control signal when the electrical characteristic measured by the electrical sensors indicates that the electrodes are aligned with a target anatomical feature.

13. A method of ablating a mammalian subject, comprising:
inserting an ablation probe into a mammalian subject, the ablation probe comprising:
an elongated body having a tapered distal end and a proximal end that are aligned along an axis;
first and second electrical sensors disposed on the elongated body; and
an electrode disposed on the elongated body between the first and second electrical sensors, wherein:
the first and second electrical sensors and the electrode are electrically coupled to a power source, and
the first and second electrical sensors have outputs that are electrically coupled to an input of a computer;
monitoring, with the computer, an electrical characteristic measured by the first and second electrical sensors;
producing, with the computer, an output control signal when the electrical characteristic measured by the first and second electrical sensors indicates that the electrode is aligned with a target anatomical feature; and
ablating the target anatomical feature, with the electrode, after the electrode is aligned with the target anatomical feature.

14. The method of claim 13, further comprising automatically applying power to the electrode in response to the output control signal.

15. The method of claim 14, further comprising automatically closing an interlock switch in response to the output control signal, the interlock switch electrically connecting the electrode to a power source when the interlock switch is in a closed state.

16. The method of claim 13, further comprising:
determining, with the computer, when a magnitude of the electrical characteristic measured by the first and second electrical sensors is higher than the magnitude of a respective baseline output signal of the first and second electrical sensors; and
producing the output control signal when the magnitude of the electrical characteristic measured by the first and second electrical sensors is higher than the magnitude of the respective baseline output signal of the first and second electrical sensors.

17. The method of claim 13, further comprising:
(a) determining, with the computer, when a magnitude of the electrical characteristic measured by the first electrical sensor is higher than the magnitude of a baseline output signal of the first electrical sensor and the magnitude of the electrical characteristic measured by the second electrical sensor is about equal to the magnitude of a baseline output signal of the second electrical sensor;
(b) after step (a), determining, with the computer, when the magnitude of the electrical characteristic measured by the first and second electrical sensors is about equal to the magnitude of the baseline output signals of the first and second electrical sensors, respectively; and
(c) producing the output control signal when the magnitude of the electrical characteristic measured by the first and second electrical sensors is about equal to the magnitude of the baseline output signals of the first and second electrical sensors, respectively,
wherein the first electrical sensor is located closer to the tapered distal end than the second electrical sensor.

18. The method of claim 17, further comprising (d) advancing the ablation probe distally in the mammalian subject between steps (a) and (b), wherein the ablation probe is in a first position in step (a) and in a second position in step (b).

19. The method of claim 18, further comprising:
(e) determining, with the computer, when the magnitude of the electrical characteristic measured by the second electrical sensor is higher than the magnitude of the baseline output signal of the second electrical sensor and the magnitude of the electrical characteristic measured by the first electrical sensor is about equal to the magnitude of the baseline output signal of the first electrical sensor;
(f) advancing the ablation probe distally in the mammalian subject between steps (b) and (e), wherein the ablation probe is in a third position in step (e), the second position between the first position and the third position; and
(g) retracting the ablation probe proximally in the mammalian subject after step (e) to a fourth position, the fourth position between the first position and the third position;
(h) after step (g), repeating step (b),
wherein step (c) occurs after step (h).

20. The method of claim 13, wherein the electrical characteristic comprises a resistance or an impedance.

21. The method of claim 13, further comprising producing a sensory output signal, with the computer, in response to the output control signal.

22. The method of claim 13, further comprising:
monitoring a measured temperature of the target anatomical feature with a thermocouple on the ablation probe, the thermocouple in electrical communication with the computer; and
stopping an ablation of the target anatomical feature when the measured temperature is higher thana predetermined ablation threshold temperature for a predetermined time period.
